# EUROPEAN PATENT APPLICATION

(11) **EP 4 088 740 A1**
(43) Date of publication of application: **16.11.2022**
(21) Application number: 21738991.5
(22) Date of filing: 05.01.2021
(51) Int. Cl.: A61K 39/395, A61K 47/12

(54) **PROGRAMMED CELL DEATH RECEPTOR 1 ANTIBODY FORMULATION AND USE THEREOF**

(30) Priority: 06.01.2020 CN 202010008058
(71) Applicant: Shanghai Henlius Biotech, Inc., Shanghai 201210 (CN)
(72) Inventor: FANG, Yuan, Shanghai 201210 (CN); HAN, Dongmei, Shanghai 201210 (CN)
(74) Representative: Keil & Schaafhausen Patentanwälte PartGmbB
(86) International application number: PCT/CN2021/070248
(87) International publication number: WO 2021/139642

(57) **Abstract**

The present invention provides a pharmaceutical formulation containing an anti-PD-1 monoclonal antibody. The pharmaceutical formulation comprises an anti-PD-1 monoclonal antibody, a citrate-sodium citrate buffer solution, a protein protectant, a surfactant, and an isotonic regulator. The present invention also relates to an application of the pharmaceutical formulation in the preparation of a liquid formulation or lyophilized formulation for injection.

## Description

### Cross-Reference to Related Applications

The present application claims priority to Chinese patent application No. 202010008058.6 filed on January 6, 2020 and entitled with "PROGRAMMED CELL DEATH RECEPTOR 1 ANTIBODY FORMULATION AND USE THEREOF", the content of which is incorporated herein by reference in its entirety.

### Technical Field

The present invention relates to the field of biotechnology, and in particular, to a programmed cell death receptor 1 (PD-1) antibody formulation and the use thereof.

### Background Art

Programmed cell death receptor 1 (PD-1), also known as CD279, is an important immunosuppressive molecule, belongs to the CD28 immunoglobulin superfamily, and is a type I transmembrane glycoprotein with a molecular weight of 50-55 kD. PD-1 is continuously expressed on the surface of T cells, B cells, natural killer cells, monocytes and bone marrow cells. There are two known ligands for PD-1, PD-L1 and PD-L2. Recently, studies have found that the binding of PD-1 to its ligand PD-L1 can transmit inhibitory signals and reduce the proliferation of CD8+ T cells in lymph nodes. Moreover, PD-1 can also control the accumulation of antigen-specific T cells in lymph nodes by regulating the Bcl-2 gene. Blocking the binding of PD-1 to PD-L1 can effectively prevent the production of inhibitory signals in T lymphocytes, thus breaking the peripheral immune tolerance of the immune system to tissues of the same body, and promoting the activation and proliferation of T lymphocytes and the expression of cytokines. Therefore, developing new anti-PD-1 monoclonal antibodies for cancer immunotherapy and endowing same with lower toxicity and side effects and better clinical efficacy have become a current research hotspot, and would also provide patients with more drug options.

Protein (such as monoclonal antibody) pharmaceutical formulations are suitable for parenteral administration, including intravenous, intramuscular, intraperitoneal or subcutaneous injection and other routes of administration. The liquid formulation is convenient to use, but has a poor stability and a higher requirement for storage conditions, and generally needs to be stored under a relatively low temperature. In addition, the protein in the liquid formulation is apt to form aggregates or particles, which will lead to reduced stability of the monoclonal antibody formulation. The proteins, especially monoclonal antibody drugs comprised in common pharmaceutical formulations are difficult to maintain good physical, chemical and biological stability during the storage period, especially under non-refrigerated conditions. Therefore, there is a need for a stable buffer solution system for such drugs. It has been reported that stabilizers that can be added to protein (monoclonal antibody) drugs comprise surfactants, sugars and polyols, salts, amino acids/amino acid salts, and some macromolecular compounds. However, antibody drugs have different structural properties and are apt to aggregate. At present, there is still an unmet need for stable protein formulations for use in the pharmaceutical industry.

### Summary of the Invention

One of the objectives of the present invention is to provide a pharmaceutical formulation containing an anti-PD-1 monoclonal antibody.

Firstly, it is determined in the present invention by means of screening and formula optimization research that the citrate-sodium citrate buffer solution is used as the preferred buffer system for the anti-PD-1 monoclonal antibody pharmaceutical formulation. Specifically, the pharmaceutical formulation comprises an anti-PD-1 monoclonal antibody, a buffer solution, a stabilizer and a surfactant, and optionally comprises an isotonic regulator.

On the basis of the screened buffer solution system, a single-factor test is designed to investigate the effects of factors such as protein concentration, pH, types and content of the stabilizer, types and content of the surfactant, and addition or absence of an isotonic regulator on the protein stability, thus obtaining the other preferred formulation components by optimization and screening.

In some embodiments, the pharmaceutical formulation comprises: an anti-PD-1 monoclonal antibody, a citrate-sodium citrate buffer solution, a protein protectant, and a surfactant.

The content of the anti-PD-1 monoclonal antibody in the pharmaceutical formulation is preferably 5-50 mg/mL, more preferably 8-30 mg/mL, and preferably 10.0 mg/mL. The anti-PD-1 monoclonal antibody optionally has a heavy chain variable region shown in SEQ ID NO. 7 and a light chain variable region shown in SEQ ID NO. 8. In a preferred embodiment, the anti-PD-1 monoclonal antibody is an h1G4 antibody, which comprises a heavy chain shown in SEQ ID NO. 10 and a light chain shown in SEQ ID NO. 9.

In one specific embodiment, the pharmaceutical formulation contains 10 mg/ml of the anti-PD-1 monoclonal antibody, wherein the anti-PD-1 monoclonal antibody is an h1G4 antibody and comprises a heavy chain shown in SEQ ID NO. 10 and a light chain shown in SEQ ID NO. 9.

The concentration of the citrate-sodium citrate is preferably 10-50 mM, more preferably 15-30 mM, and preferably 20 mM in the buffer solution system of the formulation. In one embodiment, the content of the citrate-sodium citrate buffer solution is 10-30 mM.

In one specific embodiment, the pharmaceutical formulation contains 20 mmol/L of the citrate-sodium citrate buffer solution.

The pH of the pharmaceutical formulation is preferably 5.0-6.5. For example, the pH can be 5.0, 5.5, 6.0 or 6.5. Preferably, the pH of the pharmaceutical formulation is 5.5.

A protein protectant is also added to the formulation of the present invention to provide stability to the product and is selected from conventional protein protectants in the art. The protein protectant can protect the stability of the protein drug and protect the function of the protein drug against the effects of changes in the conditions (such as changes in freezing, lyophilization, or other preparation conditions). The protein protectant preferably comprises: sugars, proteins, amino acids, polymers, salts, amines, surfactants, etc. More preferably, the protein protectant comprises: one or more of sucrose, mannitol or glycine. In one embodiment, the protein protectant is selected from one or more of sucrose, glycine and mannitol. In one specific embodiment, the protein protectant is mannitol, sucrose or glycine.

The content of the protein protectant is preferably 0.5%-5% by mass/volume (g/L). In still another embodiment, the content of the protein protectant is 1%-5% by mass/volume, wherein the mass/volume used is in g/L.

In a preferred embodiment, the protein protectant is selected from one or more of sucrose, glycine and mannitol and the content thereof is 0.5%-5% by mass/volume, wherein the mass/volume used is in g/L.

In one embodiment, the pharmaceutical formulation contains 1%-3% of sucrose by mass/volume. In still another embodiment, the pharmaceutical formulation contains 1% of glycine by mass/volume. In a preferred embodiment, the pharmaceutical formulation contains 3% of mannitol by mass/volume. The mass/volume used as described above is in g/L. The formulation of the present invention may include a surfactant, which is selected from conventional surface-active agents in the art and is preferably a nonionic surfactant. Examples of the surfactant herein preferably include: polysorbates (such as polysorbate 20 and polysorbate 80); Poloxamer (such as Poloxamer 188); Triton; sodium dodecyl sulfate (SDS); sodium lauryl sulfate; sodium octyl glycoside; lauryl-, myristyl-, linoleyl-, or stearyl-sulfobetaine; lauryl-, myristyl-, linoleyl-, or stearyl-sarcosine; linoleyl-, myristyl, or cetyl-betaine; lauramidopropyl-, cocamidopropyl-, linoleamidopropyl-, myristamidopropyl-, palmidopropyl-, or isostearamidopropyl-betaine (e.g., lauramidopropyl-betaine); myristamidopropyl-, palmidopropyl-, or isostearamidopropyl-dimethylamine; sodium methyl cocoyl-, or disodium methyl oleyl-taurate; and MONAQUAT^{™} series (Mona Industries, Inc., Paterson, N.J.); and polyethylene glycol, polypropylene glycol, and a copolymer of ethylene glycol and propylene glycol (e.g., Pluronics and PF68). In some preferred embodiments, the surfactant is polysorbate. More preferably, the surfactant is polysorbate 80.

The content of the surfactant is preferably 0.01%-0.05% by volume. In a preferred embodiment, the content of the surfactant is 0.02% by volume.

In one specific embodiment, the pharmaceutical formulation contains 0.02% of polysorbate 80 by volume.

In one specific embodiment, the pharmaceutical formulation comprises: an anti-PD-1 monoclonal antibody, a citrate-sodium citrate buffer solution, a protein protectant and a surfactant, wherein
the content of the citrate-sodium citrate buffer solution is 10-30 mM;
the protein protectant is selected from one or more of sucrose, glycine and mannitol and the content thereof is 0.5%-5% by mass/volume, wherein the mass/volume percentage is in g/L;
the surfactant is polysorbate 80 and the content thereof is 0.01%-0.05% by volume;
and the anti-PD-1 monoclonal antibody has a heavy chain variable region shown in SEQ ID NO. 7 and a light chain variable region shown in SEQ ID NO. 8.

The formulation of the present invention may or may not contain an isotonic regulator as needed, wherein the isotonic regulator is a conventional isotonic regulator in the art. In a preferred embodiment, the isotonic regulator is sodium chloride. In one embodiment, the content of the isotonic regulator is 0.1%-0.5% by mass/volume, wherein the mass/volume used is in g/L.

In one specific embodiment, the pharmaceutical formulation contains 0.3% of sodium chloride by mass/volume, wherein the mass/volume used is in g/L.

In the present invention, the formulations of various components are subjected to a high-temperature accelerated test at 40°C for up to 4 weeks to detect and evaluate the stability data of the formula of each formulation. The detection items include: appearance, concentration, turbidity (A350), pH, osmolality, purity (SEC-HPLC, CEX-HPLC) and other tests. The optimal formulation formula is obtained by means of performing an analysis on the basis of test results.

In one specific embodiment, the pharmaceutical formulation contains: 10 mg/ml of the anti-PD-1 monoclonal antibody, 20 mmol/L of the citrate-sodium citrate buffer solution, 3% of mannitol by mass/volume, 0.02% of polysorbate 80 by volume, and 0.3% of sodium chloride by mass/volume, pH 5.5, wherein the mass/volume used is in g/L.

In another specific embodiment, the pharmaceutical formulation contains: 10 mg/ml of the anti-PD-1 monoclonal antibody, 20 mmol/L of the citrate-sodium citrate buffer solution, 1%-3% of sucrose by mass/volume, 0.02% of polysorbate 80 by volume, and 0.3% of sodium chloride by mass/volume, pH 5.5, wherein the mass/volume used is in g/L.

In still another specific embodiment, the pharmaceutical formulation contains: 10 mg/ml of the anti-PD-1 monoclonal antibody, 20 mmol/L of the citrate-sodium citrate buffer solution, 1% of glycine by mass/volume, 0.02% of polysorbate 80 by volume, and 0.3% of sodium chloride by mass/volume, pH 5.5, wherein the mass/volume used is in g/L.

The dosage forms of the pharmaceutical formulation can be conventional dosage forms in the art, preferably including a liquid formulation or lyophilized formulation for injection. The liquid formulation for injection preferably includes a formulation for subcutaneous injection, a formulation for intravenous injection, a formulation for intraperitoneal administration, a formulation for intramuscular injection, a formulation for intravenous/subcutaneous injection or a formulation for intravitreal injection. The liquid formulation for injection preferably includes a formulation of aqueous solution for injection and a formulation for prefilled syringe injection, preferably the formulation of aqueous solution for injection, and the formulation of aqueous solution for injection can be used for intravenous injection. Therefore, in a further aspect, the present invention also relates to an application of the pharmaceutical formulation in the preparation of a liquid formulation or lyophilized formulation for injection.

Unless otherwise stated, the terms and phrases as used herein have the meanings listed below. A specific term or phrase shall not be considered as unclear or indefinite when it is not specifically defined, but should be interpreted according to the meanings commonly understood by those skilled in the art. When a trade name appears herein, it is intended to refer to the corresponding commodity or an active ingredient thereof. Each of the documents cited herein (including all patents, patent applications, scientific publications, manufacturer's specifications, instructions), whether supra or infra, are incorporated herein by reference in its entirety. Any content herein should not be interpreted as an admission that the present invention is not entitled to the right of priority of the earlier application.

The use of any and all embodiments, or exemplary language (e.g., "such as") provided herein is only intended to better illustrate the present invention but does not limit the scope of the present invention.

Unless otherwise stated, any numerical value, such as the concentration, concentration range, or content described herein, should be understood as being modified in all instances by the term "about". Therefore, a numerical value generally includes ± 10% of the numerical value. For example, a concentration of 1 mg/mL includes 0.9 mg/mL to 1.1 mg/mL. Likewise, a concentration range of 1% to 10% (w/v) includes 0.9% (w/v) to 11% (w/v). As used herein, unless the context clearly indicates otherwise, the use of a numerical range expressly includes all possible sub-ranges and all individual numeric values within the range, including the integers and fractions of the numerical values within the range.

The expression "comprising" or similar expressions with the same meanings "including", "containing", "having", etc. are open-ended and do not exclude additional unlisted elements, steps or ingredients. The expressions "consisting of" or "consists of" exclude any unspecified element, step or ingredient. The expression "consisting essentially of" means that the range is limited to the specified elements, steps or ingredients plus optional elements, steps or ingredients that do not substantively affect the basic and new features of the claimed subject matter. It should be understood that the expression "comprising" encompasses the expressions "consisting essentially of" and "consisting of'.

The terms "optional" or "optionally" mean that the subsequently described event or circumstance may or may not occur, and the description includes the occurrence and the non-occurrence of the event or circumstance. For example, a composition optionally comprising an isotonic regulator encompass the circumstances with or without an isotonic regulator.

The term "pharmaceutical formulation" refers to a drug to be administered to a patient in need of treatment, which formulation can be in solid or liquid form, etc. (i.e., "dosage form"), and for example can be liquid formulations (e.g., a liquid formulation for injection), powders and lyophilized formulations, etc.

The pharmaceutical formulation of the present invention shows no obvious changes in terms of main detection indicators after being subjected to the high-temperature accelerated test; and after the formulation is subjected to an accelerated treatment at 40°C for 4 weeks, it is indicated upon detection that each main detection indicator shows no obvious change and is relatively stable, and all detection results meet the standard product quality requirements currently in force, which demonstrates a good stability.

### Brief Description of the Drawings

Figure 1. Histogram of SEC fragment content (%) in accelerated stability test (40°C) of the h1G4 antibody formulation buffer system screening study.
Figure 2. Histogram of turbidity in accelerated stability test (40°C) of the h1G4 antibody formulation buffer system screening study.

### Detailed Description of Embodiments

The present invention will be further described in the following examples, which should not be construed as a limitation to the present invention.

Source of the reagents: the reagents involved in the experimental section of the present invention are all commercially available products.

### Example 1. Method for testing samples

### 1.1. Osmolality detection

20 µl of the sample and two standards with osmolality of 290 mOsmol/kg are suctioned with a pipette, and the osmolality value of the sample and standards is determined by using the Advanced Model 2020 Osmometer.

### 1.2. SEC-HPLC

The sample is analyzed by using the Agilent 1260 high-performance liquid chromatography with the TOSOH TSK G3000 (300^{∗}7.8 mm) chromatographic column, wherein the column temperature is room temperature, the flow rate is 0.5 mL/min, and the detection wavelength is 280 nm. The composition of the mobile phase is as follows: 100 mM PB, 0.5% NaCl, pH 6.8. The test sample is diluted to about 1.0 mg/mL with the mobile phase, 50 µL of the sample is injected, and the stop time is set at 30 min.

### 1.3. CEX-HPLC

The sample is analyzed by using the Agilent 1260 high-performance liquid chromatography with the Thermo ProPac WCX-10 chromatographic column (4 × 250 mm), wherein the column temperature is 35°C, the flow rate is 1.0 mL/min, and the detection wavelength is 280 nm. The elution gradient of the mobile phase is shown in Table 1. The composition of mobile phase A is as follows: CX-1 Gradient buffer A, pH 5.6, and the composition of mobile phase B is CX-1 Gradient buffer B, pH 10.2. The test sample is diluted to about 1.0 mg/mL with mobile phase A, and 20 µL of the sample is injected.

**Table 1. Elution gradient of the mobile phase**

| Time (min) | Mobile phase B% | Flow rate (mL/min) |
|---|---|---|
| 0.00 | 10.0 | 1.0 |
| 5.00 | 10.0 | 1.0 |
| 35.00 | 40.0 | 1.0 |
| 35.01 | 100.0 | 1.0 |
| 40.00 | 100.0 | 1.0 |
| 40.01 | 10.0 | 1.0 |
| 45.00 | 10.0 | 1.0 |

### 1.4. DSC

The values of Tm onset, Tm1 and Tm2 of the test sample are tested by means of the differential scanning calorimetry. The sample is diluted to 1.0 mg/ml with placebo before scanning.

### Example 2. Preparation of antibodies

The PD-1 antibody, i.e., h1G4 is derived from the h1G4 disclosed in patent application WO 2018052818, and the preparation method therefor fully refers to that of WO 2018052818. The amino acid sequences of the PD-1 antibody, h1G4 are as follows:

**Table 2. CDR sequences of h1G4**

| LCDR | LCDR1 | LCDR2 | LCDR3 |
|---|---|---|---|
| h1G4 | KASQDVTTAVA (SEQ ID NO: 1) | WASTRHT (SEQ ID NO: 2) | QQHYTIPWT (SEQ ID NO: 3) |
| HCDR | HCDR1 | HCDR2 | HCDR3 |
| h1G4 | FTFSNYGMS (SEQ ID NO: 4) | TISGGGSNIY (SEQ ID NO: 5) | VSYYYGIDF (SEQ ID NO: 6) |
| h1G4 | Heavy chain variable region (SEQ ID NO: 7) | Light chain variable region (SEQ ID NO: 8) | |
| Antibody | QVQLVESGGGLVKPGGSLRLS | DIQMTQSPSSLSASVGDRVTITCKA | |
| variable region sequences | CAASGFTFSNYGMSWIRQAP GKGLEWVSTISGGGSNIYYA DSVKGRFTISRDNAKNSLYLQ MNSLRAEDTAVYYCVSYYYG IDFWGQGTSVTVSS | | SQDVTTAVAWYQQKPGKAPKLLI YWASTRHTGVPSRFSGSGSGTDFT LTISSLQPEDFATYYCQQHYTIPW TFGGGTKLEIK |

Light chain (LC) of h1 G4 antibody SEQ ID NO: 9 
DIQMTQSPSSLSASVGDRVTITCKASQDVTTAVAWYQQKPGKAPKLLIYWASTRH
TGVPSRFSGSGSGTDFTLTISSLQPEDFATYYCQQHYTIPWTFGGGTKLEIKRTVAAPSV
FIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC
Heavy chain (HC) of h1 G4 antibody SEQ ID NO: 10

QVQLVESGGGLVKPGGSLRLSCAASGFTFSNYGMSWIRQAPGKGLEWVSTISGG
GSNIYYADSVKGRFTISRDNAKNSLYLQMNSLRAEDTAVYYCVSYYYGIDFWGQGTS
VTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPE
FLGGPSVFLFPPKPKDTLMISRTPEVTCVVVDVSQEDPEVQFNWYVDGVEVHNAKTK
PREEQFNSTYRVVSVLTVLHQDWLNGKEYKCKVSNKGLPSSIEKTISKAKGQPREPQV
YTLPPSQEEMTKNQVSLTCLVKGFYPSDIAVEWESNGQPENNYKTTPPVLDSDGSFFL
YSRLTVDKSRWQEGNVFSCSVMHEALHNHYTQKSLSLSLGK

### Example 3. Buffer system screening study

In this study, two groups of different formulation buffer systems are selected as alternative formulations (hereinafter referred to as B1-B6, respectively), and the concentration of the target drug, i.e., the h1G4 antibody is 10.0 mg/mL. B1-B3 are 20 mmol/L of citrate-sodium citrate buffer systems with the pH of 5.0, 5.5 and 6.0, respectively; B4-B6 are 20 mmol/L of histidine-histidine hydrochloride buffer systems with the pH of 5.0, 5.5 and 6.0, respectively; the above-mentioned buffer systems all contain 3% of mannitol, 0.02% of polysorbate 80 and 0.3% of sodium chloride as auxiliary materials, wherein the concentration percentages of the auxiliary materials refer to the mass/volume used (w/v, g/L). The information of alternative formulations of the h1G4 antibody formulation buffer system screening study is shown in Table 3.

**Table 3. Composition of alternative buffer system of h1 G4 antibody formulation**

| **Formula tion No.** | **Formula tion code** | **Buffer solution type and ionic strength** | **pH** | **Mannitol content (%)** | **Sodium chloride content (%)** | **Polysorbate 80 content (%)** |
|---|---|---|---|---|---|---|
| B1 | C50 | 20 mmol/L Citrate-sodium citrate | 5.0 | 3 | 0.3 | 0.02 |
| B2 | C55 | | 5.5 | 3 | 0.3 | 0.02 |
| B3 | C60 | | 6.0 | 3 | 0.3 | 0.02 |
| B4 | H50 | 20 mmol/L histidine-histidine hydrochloride | 5.0 | 3 | 0.3 | 0.02 |
| B5 | H55 | | 5.5 | 3 | 0.3 | 0.02 |
| B6 | H60 | | 6.0 | 3 | 0.3 | 0.02 |

An appropriate amount of the antibody stock solution is taken and subjected to ultrafiltration and medium exchange, auxiliary materials are added, and the concentration is adjusted to prepare the formulations as shown in Table 3. In a biosafety cabinet, the sample is filtered by using a 0.22 µm disposable sterile filter and then aseptically sub-packaged into 2 mL penicillin vials. The vials are capped with 13 mm rubber stoppers, and 13 mm aluminum-plastic composite caps are used to seal the stoppers. The sub-packaged samples are placed uprightly in a constant temperature medicinal storage box at 40°C and 2°C-8°C for 2 weeks, the samples are taken at week 0, 1, and 2, respectively for detection and analysis, and the details of the test items can be seen in Table 4.

**Table 4. Examination conditions of the h1G4 antibody buffer system screening study**

| Examination conditions | Detection time point | | |
|---|---|---|---|
| | Week 0 | Week 1 | Week 2 |
| 40°C | X, Y | X | X |
| 2°C-8°C | | | X |
| X = appearance, concentrat tion, A₃₅₀, SEC-HPLC , CEX-HPLC, and R-CE-SDS | | | |
| Y = osmolality, and DSC; -- indicates that the sa mple is not detected at this time point. | | | |

### Research results

### High-temperature accelerated test results

At week 0, particles appear in the 20 mmol/L pH 5.0 citrate system (C50); and the appearances of the other buffer systems are colorless and slightly opalescent liquid, without obvious visible particles. The concentration is within the range of 9-11 mg/mL, and the osmolality is 300-340 mOsmol/kg. The contents of SEC main peaks are all greater than 99.0%, and the content of the aggregates is 0.6%-0.7%. The contents of CEX main peaks are 47.0%-48.6%, the contents of the acid peaks are 11.0%-14.3%, the contents of the basic peaks are 38.6%-40.9%, and the purity of R-CE-SDS is 94.1%-96.9%. The basic physical and chemical detection results show that at week 0, the quality of each buffer system is good, showing no significant difference (see Table 5).

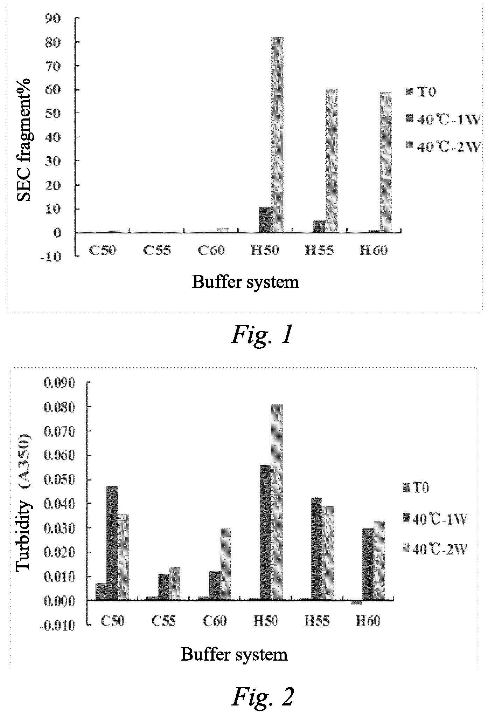

After being placed under accelerated conditions at 40°C for 2 weeks, the concentrations of proteins in all citrate-sodium citrate buffer systems show no obvious change and are within the range of 9-11 mg/mL. However, the concentrations in the histidine-histidine hydrochloride buffer systems increase significantly, and protein precipitates appear in the C50, C60 and H55 buffer systems. The measurements of turbidity in the histidine-histidine hydrochloride buffer system (A350 is tested by using NanoDrop 2000C, 2.5 µL of sample is injected, and the absorbance value of the sample at the wavelength of 350 nm is measured. The measurement is repeated three times and the average value is taken as the final result.) increase obviously (see Figure 1 and Figure 2). Hence, the buffer systems are ranked from superiority to inferiority as C55>C60>C50, H60>H55>H50. After being placed at 40°C ± 2°C for 2 weeks, it is found by the CE-SDS determination that the content of the heavy chains is decreased by 5.4%-23.7%, and the systems are ranked from superiority to inferiority according to the purity as C55, H55, H50>H60>C50>C60.

After being placed under accelerated conditions at 40°C for 2 weeks, the content of the SEC main peaks of the citrate-sodium citrate buffer system is decreased by 0.1%-2.5%, the content of the aggregates is increased by 0.1%-0.4%, and the content of the fragments is increased by 0%-2.1%. However, all the histidine-histidine hydrochloride buffer systems have obvious degradation reaction, and therefore, the citrate-sodium citrate buffer systems are better than the histidine-histidine hydrochloride systems, and the optimum pH is 5.5.

**Table 5. Data summary table of accelerated stability test (40°C) of the h1G4 antibody buffer system screening study**

| **Formul ation No.** | **Formu lation code** | **Sampli ng time point** | **Appearance** | **Concent ration (mg/mL)** | **Turbid ity A₃₅₀** | **SEC-HPLC** | | | **CEX-HPLC** | | | **R-CE-SDS** | | |
|---|---|---|---|---|---|---|---|---|---|---|---|---|---|---|
| | | | | | | **Main peak%** | **Aggre gate%** | **Fragm ent%** | **Main peak %** | **Acid peak %** | **Basic peak%** | **Light chain %** | **Heavy chain %** | **Purity %** |
| **B1** | **C50** | **0** | Colorless and slightly opalescent | 9.8 | 0.008 | 99.3 | 0.7 | 0.0 | 47.0 | 13.8 | 39.2 | 31.1 | 65.8 | **96.9** |
| | | Week 1 | Colorless and slightly opalescent, with particles | 9.7 | 0.048 | 99.0 | 0.9 | 0.1 | 43.5 | 26.7 | 29.8 | N/A | N/A | N/A |
| | | Week 2 | Colorless emulsion, protein precipitates | 9.8 | 0.036 | 98.3 | 0.8 | 0.9 | 34.7 | 43.9 | 21.4 | 31.7 | 43.4 | 75.1 |
| B2 | C55 | 0 | Colorless and slightly opalescent | 10.1 | 0.002 | 99.4 | 0.7 | 0.0 | 47.7 | 13.4 | 39.0 | 30.3 | 66.1 | 96.4 |
| | | Week 1 | Colorless and slightly opalescent | 10.5 | 0.011 | 99.1 | 0.8 | 0.1 | 47.7 | 15.1 | 37.1 | N/A | N/A | N/A |
| | | Week 2 | Colorless microemulsion | 10.5 | 0.014 | 99.2 | 0.8 | 0.0 | 43.4 | 23.9 | 32.7 | 30.6 | 60.7 | 91.2 |
| B3 | C60 | 0 | Colorless and slightly opalescent | 9.9 | 0.002 | 99.4 | 0.7 | 0.0 | 47.1 | 14.3 | 38.6 | 30.4 | 63.7 | 94.1 |
| | | Week | Colorless and 1 slightly opalescent | 10.0 | 0.012 | 99.0 | 0.9 | 0.1 | 41.2 | 33.1 | 25.7 | N/A | N/A | N/A |
| | | Week 2 | Colorless emulsion, 2 protein precipitates | 10.2 | 0.030 | 96.9 | 1.0 | 2.1 | 30.9 | 50.5 | 18.7 | 31.7 | 40.0 | 71.7 |
| B4 | H50 | 0 | Colorless and slightly opalescent | 9.5 | 0.001 | 99.3 | 0.7 | 0.0 | 48.6 | 11.0 | 40.4 | 30.4 | 65.1 | 95.4 |
| | | Week | Colorless 1 microemulsion | 17.8 | 0.056 | 87.9 | 1.2 | 10.9 | 48.7 | 13.3 | 38.0 | N/A | N/A | N/A |
| | | Week | 2 Colorless emulsion, 2 protein precipitates | 43.0 | 0.081 | 17.8 | 0.1 | 82.1 | 47.8 | 11.7 | 40.5 | 39.3 | 52.0 | 91.3 |
| B5 | H55 | 0 | Colorless and slightly opalescent | 9.9 | 0.001 | 99.3 | 0.7 | 0.0 | 48.2 | 11.0 | 40.9 | 30.9 | 64.0 | 95.0 |
| | | Week 1 | Colorless microemulsion | 14.4 | 0.043 | 94.0 | 1.0 | 5.0 | 47.3 | 14.2 | 38.5 | N/A | N/A | N/A |
| | | Week 2 | Colorless microemulsion | 22.7 | 0.039 | 39.0 | 0.4 | 60.5 | 45.7 | 17.4 | 37.0 | 34.3 | 56.8 | 91.1 |
| B6 | H60 | 0 | Colorless and slightly opalescent | 10.2 | -0.002 | 99.4 | 0.7 | 0.0 | 47.6 | 11.5 | 40.9 | 29.9 | 64.8 | 94.7 |
| | | Week 1 | Colorless microemulsion | 12.4 | 0.030 | 98.0 | 0.9 | 1.1 | 46.6 | 16.4 | 37.0 | N/A | N/A | N/A |
| | | Week 2 | Colorless microemulsion | 23.8 | 0.033 | 40.6 | 0.3 | 59.0 | 42.8 | 22.3 | 34.9 | 31.1 | 55.3 | 86.5 |

| | | | | | | | | | | | | | | |
|---|---|---|---|---|---|---|---|---|---|---|---|---|---|---|
| Note: N/A indicates not applicable. | | | | | | | | | | | | | | |

### Example 4. Auxiliary material screening study

In this round of study, there are 6 alternative formulations (hereinafter referred to as F1-F6, respectively), and the concentration of the target protein, i.e., the h1G4 antibody is 10.0 mg/mL. The buffer systems are all 20 mmol/L of citrate-sodium citrate solutions (pH 5.5). The formulation F3 does not contain sodium chloride, and the other formulations contain 0.3% of sodium chloride and 0.02% of polysorbate 80, wherein the content of mannitol in formulations F1, F2 and F4 is 1%, 3% and 5%, respectively. The effect of the content of mannitol on the protein stability is investigated by using a single factor test. Formulations F2 (0.3% of NaCl) and F3 (0% of NaCl) are compared to investigate whether sodium chloride facilitates protein stability. The formulations of F2, F5 and F6 respectively contain 3% of mannitol, 3% of sucrose, and 1% of glycine (the three of which are isotonic) for the purpose of screening the optimal auxiliary material system contributing to the long-term stability of the h1G4 antibody formulations. The information of alternative formulations of the h1G4 antibody formulation auxiliary material screening study can be seen in Table 6.

**Table 6. The composition of alternative formulations of the h1G4 antibody formulation auxiliary material screening study**

| | | | | | |
|---|---|---|---|---|---|
| | | | | | |

| Formulation No. | Buffer solution type | pH | Stabilizer (%) | Sodium chloride content (%) | Polysorbate 80 content |
|---|---|---|---|---|---|
| | and ionic strength | | | | (%) |
| F1 | 20 mmol/L Citrate-sodium citrate | 5.5 | 1% of mannitol | 0.3 | 0.02 |
| F2 | | | 3% of mannitol | 0.3 | 0.02 |
| F3 | | | 3% of mannitol | 0.0 | 0.02 |
| F4 | | | 5% of mannitol | 0.3 | 0.02 |
| F5 | | | 3% of sucrose | 0.3 | 0.02 |
| F6 | | | 1% of glycine | 0.3 | 0.02 |

An appropriate amount of the stock solution is taken and subjected to ultrafiltration and medium exchange, auxiliary materials are added, and the concentration is adjusted to prepare the formulations as shown in Table 6. In a biosafety cabinet, the sample is filtered by using a 0.22 µm disposable sterile filter and then aseptically sub-packaged into 2 mL penicillin vials. The vials are capped with 13 mm rubber stoppers, and 13 mm aluminum-plastic composite caps are used to seal the stoppers. The sub-packaged samples are placed uprightly in a constant temperature medicinal storage box at 40°C and 2°C-8°C for 8 weeks. The samples are taken and detected at week 0, 1, 2, 4, 6 and 8, respectively.

In the single-factor test for the h1G4 antibody formulation auxiliary material screening study, 6 formulations are designed. According to the 40°C accelerated stability test results and the rank from superiority to inferiority of the auxiliary materials (see Table 7), the formulation without sodium chloride (F3) and the formulation containing 5% of mannitol (F4) have slightly poor stability, and there is no significant difference among the formulations containing 1% of mannitol (F1), 3% of mannitol (F2) and the formulation containing 3% of sucrose (F5).

**Table 7. Data summary table of high-temperature accelerated test (40°C) of the h1G4 antibody formulation auxiliary material screening study**

| **Formu lation No.** | **Sampli ng time point** | **Appearance** | **Concentr ation mg/mL** | **Osmolality mOsm/kg** | **A₃₅₀** | **SEC-HPLC** | | | **CEX-HPLC** | | |
|---|---|---|---|---|---|---|---|---|---|---|---|
| | | | | | | **Main peak%** | **High polymer %** | **Fragm ent%** | **Main peak %** | **Acid peak%** | **Basic peak%** |
| F1 | 0 | Colorless and slightly opalescent | 10.3 | 207 | 0.029 | 96.6 | 3.4 | 0.0 | 39.2 | 26.6 | 34.3 |
| | Week 1 | Colorless and slightly opalescent | 10.2 | N/A | 0.008 | 96.8 | 3.1 | 0.1 | 41.6 | 29.1 | 29.3 |
| | Week 2 | Colorless and slightly opalescent | 10.3 | 206 | 0.008 | 96.3 | 3.5 | 0.2 | 36.7 | 33.5 | 29.8 |
| | Week 4 | Colorless and slightly opalescent | 9.7 | N/A | 0.004 | 95.0 | 4.2 | 0.8 | 31.5 | 40.9 | 27.6 |
| | Week 6 | Colorless and slightly opalescent | 10.6 | N/A | 0.008 | 94.8 | 4.2 | 0.9 | 25.0 | 47.4 | 27.6 |
| | Week 8 | Colorless and slightly opalescent | 10.5 | N/A | 0.005 | 95.1 | 3.5 | 1.4 | 23.2 | 52.9 | 23.9 |
| F2 | 0 | Colorless and slightly opalescent | 10.4 | 326 | 0.024 | 96.7 | 3.3 | 0.0 | 39.3 | 26.6 | 34.1 |
| | Week 1 | Colorless and slightly opalescent | 10.3 | N/A | 0.006 | 96.9 | 3.0 | 0.1 | 40.4 | 29.1 | 30.5 |
| | Week 2 | Colorless and slightly opalescent | 10.5 | 322 | 0.008 | 96.3 | 3.6 | 0.2 | 37.1 | 33.6 | 29.4 |
| | Week 4 | Colorless and slightly opalescent | 9.8 | N/A | 0.007 | 95.1 | 4.1 | 0.8 | 30.8 | 40.1 | 29.1 |
| | Week 6 | Colorless and slightly opalescent | 10.7 | N/A | 0.007 | 95.0 | 4.1 | 1.0 | 24.0 | 47.1 | 28.9 |
| | Week 8 | Colorless and slightly opalescent | 10.8 | N/A | 0.004 | 95.0 | 3.6 | 1.5 | 23.1 | 52.5 | 24.4 |
| F3 | 0 | Colorless and slightly opalescent | 10.7 | 230 | 0.037 | 96.7 | 3.3 | 0.0 | 39.2 | 26.9 | 33.9 |
| | Week 1 | Colorless and slightly opalescent | 10.5 | N/A | 0.007 | 96.9 | 3.1 | 0.1 | 41.2 | 30.1 | 28.7 |
| | Week 2 | Colorless and slightly opalescent | 10.6 | 230 | 0.007 | 96.3 | 3.6 | 0.2 | 36.4 | 34.9 | 28.8 |
| | Week 4 | Colorless and slightly opalescent | 10.0 | N/A | 0.006 | 95.0 | 4.3 | 0.7 | 29.9 | 43.1 | 27.1 |
| | Week 6 | Colorless and slightly opalescent | 10.8 | N/A | 0.005 | 94.5 | 4.1 | 1.4 | 23.0 | 49.1 | 27.9 |
| | Week 8 | Colorless and slightly opalescent | 10.8 | N/A | 0.003 | 94.7 | 4.0 | 1.3 | 21.7 | 56.1 | 22.2 |
| F4^{∗} | 0 | Colorless and slightly opalescent | 10.3 | 442 | 0.027 | 96.7 | 3.3 | 0.0 | 38.7 | 26.7 | 34.6 |
| | Week 1 | Colorless and slightly opalescent | 10.0 | N/A | 0.006 | 97.0 | 3.0 | 0.1 | 41.5 | 29.1 | 29.4 |
| | Week 2 | Colorless and slightly opalescent | 10.1 | 431 | 0.009 | 96.5 | 3.4 | 0.1 | 36.0 | 33.7 | 30.3 |
| | Week 4 | Colorless and slightly opalescent | 9.5 | N/A | 0.006 | 95.2 | 4.0 | 0.8 | 31.1 | 40.6 | 28.3 |
| | Week 6 | Colorless and slightly opalescent | 10.3 | N/A | 0.005 | 95.1 | 3.9 | 1.0 | 24.9 | 48.7 | 26.4 |
| | Week 8 | Colorless and slightly opalescent | 10.4 | N/A | 0.008 | 94.5 | 3.8 | 1.7 | 22.1 | 55.5 | 22.4 |
| F5 | 0 | Colorless and slightly opalescent | 10.7 | 251 | 0.025 | 96.7 | 3.3 | 0.0 | 38.4 | 26.7 | 34.9 |
| | Week 1 | Colorless and slightly opalescent | 10.4 | N/A | 0.007 | 96.9 | 3.0 | 0.1 | 41.9 | 29.2 | 28.9 |
| | Week 2 | Colorless and slightly opalescent | 10.3 | 245 | 0.008 | 96.4 | 3.4 | 0.2 | 36.5 | 33.9 | 29.6 |
| | Week 4 | Colorless and slightly opalescent | 9.8 | N/A | 0.005 | 95.4 | 4.0 | 0.5 | 30.9 | 41.0 | 28.1 |
| | Week 6 | Colorless and slightly opalescent | 10.6 | N/A | 0.004 | 95.0 | 4.0 | 1.0 | 24.7 | 49.0 | 26.4 |
| | Week 8 | Colorless and slightly opalescent | 10.4 | N/A | 0.005 | 94.8 | 3.8 | 1.4 | 22.9 | 53.3 | 23.8 |
| F6 | 0 | Colorless and slightly opalescent | 10.6 | 284 | 0.005 | 96.7 | 3.3 | 0.0 | 38.1 | 27.3 | 34.7 |
| | Week 1 | Colorless and slightly opalescent | 10.4 | N/A | 0.006 | 97.2 | 2.7 | 0.1 | 41.5 | 28.9 | 29.6 |
| | Week 2 | Colorless and slightly opalescent | 10.6 | 276 | 0.006 | 96.6 | 3.2 | 0.2 | 37.1 | 33.0 | 30.0 |
| | Week 4 | Colorless and slightly opalescent | 9.7 | N/A | 0.004 | 94.9 | 4.2 | 0.9 | 32.0 | 38.4 | 29.5 |
| | Week 6 | Colorless and slightly opalescent | 10.5 | N/A | 0.007 | 94.4 | 4.2 | 1.5 | 24.9 | 44.2 | 30.9 |
| | Week 8 | Colorless and slightly opalescent | 10.5 | N/A | 0.005 | 94.3 | 3.8 | 1.8 | 24.6 | 49.3 | 26.1 |

| | | | | | | | | | | | |
|---|---|---|---|---|---|---|---|---|---|---|---|
| Note: N/A indicates not applicable. ^{∗} indicates that after being placed at 40°C ± 2°C for 2 weeks, the opalescent phenomenon of the F4 formulation is slightly more obvious than that of the other formulations. | | | | | | | | | | | |

### Example 5. Screening of stabilizer

In this round of study, the formulation formulations are screened by using a single-factor test, and a total of 9 alternative formulations (hereinafter referred to as F7-F15, respectively) are designed, and the concentration of the target drug, i.e., the h1G4 antibody is 10.0 mg/mL. The buffer systems are 20 mmol/L of citrate-sodium citrate (pH 5.5) in all the formulations of F7-F14, and 20 mmol/L of sodium citrate-disodium hydrogen phosphate in the formulation F15, and the information of the alternative formulations of the formulation auxiliary material screening study can be seen in Table 8.

**Table 8. The composition of alternative formulations of the h1G4 antibody formulation auxiliary material screening study**

| Formulation No. | Buffer solution type and ionic strength | pH | Non-reducing sugar and content (%) | Sodium chloride content (%) | Polysorbate 80 content (%) |
|---|---|---|---|---|---|
| F7 | 20 mmol/L Citrate-sodium citrate | 5.5 | 1% of mannitol | 0.3% | 0.02% |
| F8 | | | 3% of mannitol | 0.3% | 0.02% |
| F9 | | | 1% of sucrose | 0.3% | 0.02% |
| F10 | | | 3% of sucrose | 0.3% | 0.00% |
| F11 | | | 3% of sucrose | 0.3% | 0.02% |
| F12 | | | 3% of sucrose | 0.3% | 0.05% |
| F13 | | | 1% of glycine | 0.3% | 0.02% |
| F14 | | | 3% of glycine | 0.3% | 0.02% |
| F15 | 20 mmol/L Sodium citrate-disodium hydrogen phosphate | 5.5 | 1% of glycine | 0.3% | 0.02% |

An appropriate amount of the stock solution is taken and subjected to ultrafiltration and medium exchange, auxiliary materials are added, and the concentration is adjusted to prepare the formulations of Table 8. In a biosafety cabinet, the sample is filtered by using a 0.22 µm disposable sterile filter and then aseptically sub-packaged into 2 mL penicillin vials. The vials are capped with 13 mm rubber stoppers, and 13 mm aluminum-plastic composite caps are used to seal the stoppers. The sub-packaged samples are placed in a constant temperature shaker, and shaken at 200 rpm for 4 weeks at 40°C. The samples are taken, detected and analyzed at week 0, 1, 2 and 4, respectively (see Table 9).

**Table 9. Examination conditions of the h1G4 antibody formulation auxiliary material screening study**

| **Examination conditions** | **Detection time point** | | | |
|---|---|---|---|---|
| | **Week 0** | **Week 1** | **Week 2** | **Week 4** |
| 40°C, 200 rpm | X, Y | X | X | X |
| X = appearance, concentration, turbidity (A₃₅₀), pH, SEC-HPLC, and CEX-HPLC; | | | | |
| Y = osmolality | | | | |

### Research results

At week 0, all the formulations are colorless, in the form of clear liquid, with no obvious visible particles. The concentration is within the range of 9-11 mg/mL, the osmolality is 185-540 mOsmol/kg, and the turbidity (A350) is 0.006-0.009. The contents of SEC main peaks are 98.0%-98.2%, and the content of the aggregates is 1.7%-1.9%. The contents of CEX main peaks are 35.4%-39.1%, the contents of the acid peaks are 31.6%-33.8%, and the contents of the basic peaks are 27.5%-30.8% (see Table 10). The basic physical and chemical detection results show that at week 0, the quality of each formulation is good, showing no significant difference.

It can be seen after an accelerated treatment at 40°C for up to 4 weeks that the sodium citrate-disodium hydrogen phosphate buffer system is not suitable for the stable storage of the h1 G4 antibody.

**Table 10. Shaking stability test (40°C, 200 rpm) of the h1G4 antibody formulation auxiliary material screening study Data summary table**

| **Formul ation No.** | **Sampling time point** | **Appearance** | **Concent ration (mg/mL)** | **pH** | **Osmolality (mOsm/kg)** | **A₃₅₀** | **SEC-HPLC** | | | **C EX-HPLC** | | |
|---|---|---|---|---|---|---|---|---|---|---|---|---|
| | | | | | | | **Main peak%** | **High polym er%** | **Fragm ent%** | **Main peak%** | **Acid peak%** | **Basic peak** % |
| F7 | 0 | Colorless and clear | 10.2 | 5.5 | 204 | 0.007 | 98.0 | 1.9 | 0.1 | 39.1 | 31.6 | 29.3 |
| | Week 1 | Colorless and clear | 10.2 | 5.4 | N/A | 0.007 | 97.8 | 2.0 | 0.2 | 43.3 | 29.9 | 26.8 |
| | Week 2 | Colorless and clear | 10.3 | N/A | N/A | 0.003 | 97.1 | 2.2 | 0.8 | 39.8 | 33.5 | 26.7 |
| | Week 4 | Colorless and clear | 10.6 | 5.5 | N/A | 0.008 | 96.5 | 2.6 | 0.9 | 36.4 | 38.1 | 25.5 |
| F8 | 0 | Colorless and clear | 10.5 | 5.5 | 322 | 0.008 | 98.0 | 1.9 | 0.1 | 39.3 | 32.2 | 28.5 |
| | Week 1 | Colorless and clear | 10.3 | 5.5 | N/A | 0.005 | 97.8 | 2.0 | 0.1 | 44.3 | 28.5 | 27.2 |

| **Formul ation No.** | **Sampling time point** | **Appearance** | **Concent ration (mg/mL** | **pH** | **Osmolality (mOsm/kg)** | **A₃₅₀** | **SE C-HPLC** | | | **C EX-HPL C** | | |
|---|---|---|---|---|---|---|---|---|---|---|---|---|
| | | | | | | | **Main peak%** | **High polym er%** | **Fragm ent%** | **Main peak%** | **Acid peak%** | **Basic peak %** |
| | Week 2 | Colorless and clear | 10.5 | N/A | N/A | 0.004 | 97.6 | 2.2 | 0.2 | 40.8 | 32.0 | 27.2 |
| | Week 4 | Colorless and clear | 10.5 | 5.4 | N/A | 0.008 | 96.8 | 2.5 | 0.7 | 37.7 | 35.4 | 27.0 |
| F9 | 0 | Colorless and clear | 10.4 | 5.5 | 185 | 0.006 | 98.1 | 1.9 | 0.0 | 39.6 | 32.9 | 27.5 |
| | Week 1 | Colorless and clear | 10.4 | 5.5 | N/A | 0.005 | 97.8 | 2.1 | 0.1 | 44.5 | 28.6 | 26.8 |
| | Week 2 | Colorless and clear | 10.4 | N/A | N/A | 0.004 | 97.5 | 2.3 | 0.3 | 41.0 | 31.8 | 27.3 |
| | Week 4 | Colorless and clear | 10.6 | 5.5 | N/A | 0.012 | 96.7 | 2.6 | 0.7 | 37.7 | 35.7 | 26.7 |
| F10 | 0 | Colorless and clear | 10.5 | 5.5 | 242 | 0.008 | 98.2 | 1.7 | 0.1 | 37.4 | 32.6 | 30.0 |
| | Week 1 | Colorless and clear | 10.3 | 5.5 | N/A | 0.004 | 97.9 | 2.0 | 0.2 | 44.5 | 28.4 | 27.0 |
| | Week 2 | Colorless and clear, with particles | 10.4 | N/A | N/A | 0.006 | 97.6 | 2.1 | 0.3 | 41.0 | 31.8 | 27.2 |
| | Week 4 | Colorless and clear, with particles | 11.0 | 5.6 | N/A | 0.010 | 97.0 | 2.4 | 0.6 | 38.6 | 35.7 | 25.7 |
| Fll | 0 | Colorless and clear | 10.3 | 5.5 | 246 | 0.009 | 98.0 | 1.9 | 0.1 | 35.4 | 33.9 | 30.8 |
| | Week 1 | Colorless and clear | 10.4 | 5.5 | N/A | 0.005 | 97.9 | 2.0 | 0.1 | 44.5 | 28.4 | 27.1 |
| | Week 2 | Colorless and clear | 10.5 | N/A | N/A | 0.004 | 97.6 | 2.2 | 0.2 | 41.1 | 31.7 | 27.3 |
| | Week 4 | Colorless and clear | 10.6 | 5.5 | N/A | 0.006 | 96.8 | 2.5 | 0.7 | 38.7 | 35.5 | 25.9 |
| F12 | 0 | Colorless and clear | 10.3 | 5.5 | 250 | 0.006 | 98.1 | 1.9 | 0.1 | 38.9 | 33.7 | 27.4 |
| | Week 1 | Colorless and slightly opalescent | 10.3 | 5.6 | N/A | 0.007 | 97.5 | 2.3 | 0.1 | 44.5 | 28.2 | 27.3 |
| | Week 2 | Colorless and slightly opalescent | 10.4 | N/A | N/A | 0.007 | 97.6 | 2.2 | 0.2 | 41.1 | 31.6 | 27.3 |
| | Week 4 | Colorless and slightly opalescent | 10.6 | 5.5 | N/A | 0.010 | 96.8 | 2.6 | 0.6 | 38.6 | 35.6 | 25.9 |
| F13 | 0 | Colorless and clear | 10.4 | 5.5 | 298 | 0.008 | 98.2 | 1.8 | 0.1 | 37.3 | 32.6 | 30.1 |
| | Week 1 | Colorless and clear | 10.4 | 5.5 | N/A | 0.006 | 98.2 | 1.6 | 0.2 | 44.3 | 28.9 | 26.8 |
| | Week 2 | Colorless and clear | 10.5 | N/A | N/A | 0.006 | 98.0 | 1.7 | 0.2 | 41.3 | 31.4 | 27.3 |
| | Week 4 | Colorless and clear | 10.5 | 5.5 | N/A | 0.008 | 97.2 | 2.1 | 0.7 | 39.4 | 34.1 | 26.5 |
| F14 | 0 | Colorless and clear | 10.2 | 5.5 | 540 | 0.008 | 98.2 | 1.8 | 0.1 | 38.7 | 33.1 | 28.2 |
| | Week 1 | Colorless and slightly opalescent | 10.3 | 5.5 | N/A | 0.005 | 98.3 | 1.5 | 0.1 | 43.4 | 30.0 | 26.6 |
| | Week 2 | Colorless and slightly opalescent | 10.3 | N/A | N/A | 0.009 | 98.2 | 1.6 | 0.3 | 40.7 | 33.0 | 26.4 |
| | Week 4 | Colorless and slightly opalescent | 10.3 | 5.5 | N/A | 0.007 | 97.3 | 2.0 | 0.8 | 37.3 | 36.1 | 26.5 |
| F15 | 0 | Colorless and clear | 10.0 | 5.6 | 272 | 0.006 | 98.1 | 1.8 | 0.1 | 36.7 | 33.6 | 29.8 |
| | Week 1 | Colorless and clear | 10.1 | 5.4 | N/A | 0.004 | 97.7 | 1.6 | 0.7 | 40.3 | 35.6 | 24.1 |
| | Week 2 | Colorless and clear | 10.2 | N/A | N/A | 0.003 | 97.3 | 1.7 | 0.9 | 35.1 | 40.0 | 24.9 |
| | Week 4 | Colorless and clear | 10.3 | 5.6 | N/A | 0.005 | 96.2 | 2.1 | 1.7 | 30.3 | 45.6 | 24.2 |

| | | | | | | | | | | | | |
|---|---|---|---|---|---|---|---|---|---|---|---|---|
| Note: N/A indicates not applicable. | | | | | | | | | | | | |

The results indicate that after the finished products of the h1G4 formulations of the present invention are stored at 40°C for up to 4 weeks, no obvious changes can be seen in the appearance, visible particles, protein content, SEC, CEX, pH, and osmolality, all of which meet the requirements in the quality standard draft (currently in force) for the present product. It indicates that the formulations have a good stability.

It should be understood that after reading the above content of the present invention, a person skilled in the art can make various changes or modifications to the present invention, and these equivalent forms also fall within the scope defined by the appended claims of the present application.

## Claims

1. A pharmaceutical formulation of an anti-PD-1 monoclonal antibody, wherein the pharmaceutical formulation comprises an anti-PD-1 monoclonal antibody, a citrate-sodium citrate buffer solution, a protein protectant, and a surfactant, wherein
the content of the citrate-sodium citrate buffer solution is 10-30 mM;
the protein protectant is selected from one or more of sucrose, glycine and mannitol and the content thereof is 0.5%-5% by mass/volume, wherein the mass/volume used is in g/L;
the surfactant is polysorbate 80 and the content thereof is 0.01%-0.05% by volume;
and the anti-PD-1 monoclonal antibody has a heavy chain variable region shown in SEQ ID NO. 7 and a light chain variable region shown in SEQ ID NO. 8.

2. The pharmaceutical formulation according to claim 1, wherein the pharmaceutical formulation further contains sodium chloride as an isotonic regulator, and the content thereof is 0.1%-0.5% by mass/volume, wherein the mass/volume used is in g/L.

3. The pharmaceutical formulation according to claim 1 or 2, wherein the pH of the pharmaceutical formulation is 5.0-6.5.

4. The pharmaceutical formulation according to any one of claims 1-3, wherein the protein protectant is mannitol or sucrose and the content thereof is 1%-5% by mass/volume, wherein the mass/volume used is in g/L.

5. The pharmaceutical formulation according to any one of claims 1-4, wherein the protein concentration of the anti-PD-1 monoclonal antibody is 5-50 mg/ml.

6. The pharmaceutical formulation according to any one of claims 1-5, wherein the anti-PD-1 monoclonal antibody is an h1G4 antibody, which comprises a heavy chain shown in SEQ ID NO. 10 and a light chain shown in SEQ ID NO. 9.

7. The pharmaceutical formulation according to any one of claims 1-6, wherein the pharmaceutical formulation contains 10 mg/ml of the anti-PD-1 monoclonal antibody, 20 mmol/L of the citrate-sodium citrate buffer solution, 3% of mannitol by mass/volume, 0.02% of polysorbate 80 by volume, and 0.3% of sodium chloride by mass/volume, pH 5.5, wherein the mass/volume used is in g/L.

8. The pharmaceutical formulation according to any one of claims 1-6, wherein the pharmaceutical formulation contains 10 mg/ml of the anti-PD-1 monoclonal antibody, 20 mmol/L of the citrate-sodium citrate buffer solution, 1%-3% of sucrose by mass/volume, 0.02% of polysorbate 80 by volume, and 0.3% of sodium chloride by mass/volume, pH 5.5, wherein the mass/volume used is in g/L.

9. The pharmaceutical formulation according to any one of claims 1-6, wherein the pharmaceutical formulation contains 10 mg/ml of the anti-PD-1 monoclonal antibody, 20 mmol/L of the citrate-sodium citrate buffer solution, 1% of glycine by mass/volume, 0.02% of polysorbate 80 by volume, and 0.3% of sodium chloride by mass/volume, pH 5.5, wherein the mass/volume used is in g/L.

10. Use of the pharmaceutical formulation according to any one of claims 1-9 in the preparation of a liquid formulation or a lyophilized formulation for injection.
